# EUROPEAN PATENT APPLICATION

(11) **EP 4 147 742 A2**
(43) Date of publication of application: **15.03.2023**
(21) Application number: 22190491.5
(22) Date of filing: 16.08.2022
(51) Int. Cl.: A61M 25/01, A61B 5/00

(54) **SHEATH AND CATHETER HAVING MEANS FOR CONTROLLING RADIAL ORIENTATION THEREOF**

(30) Priority: 17.08.2021 US 202162233861 P; 18.07.2022 US 202217866761
(71) Applicant: Biosense Webster (Israel) Ltd, Yokneam, 2066717 (IL)
(72) Inventor: SUAREZ, Paul, Irvine, 92618 (US); LOVEJOY, Erica, Irvine, 92618 (US)
(74) Representative: Carpmaels & Ransford LLP

(57) **Abstract**

A catheter (100) including: an elongated body (106) sized to traverse vasculature, defining a longitudinal axis, and including: an outer surface; and a proximal end and a distal end disposed on opposite sides thereof; an orientation lumen (116) formed in the elongated body and defining a curved path extending proximally from the distal end towards the proximal end and about the longitudinal axis; an orientation insert (120) disposed within the orientation lumen, the orientation insert including an oriented pull-wire attached to the distal end of the elongated body such that tightening the oriented pull-wire deflects the distal end to form a curve; and a flexible circuit (130) disposed on the outer surface of the elongated body, the elongated body being substantially rotationally fixed about the longitudinal axis relative to the oriented pull-wire such that the flexible circuit is disposed toward an outer bend of the curve when the oriented pull-wire is tightened.

## Description

### CROSS REFERENCE TO RELATED APPLICATION

This application claims the benefit of priority under 35 U.S.C. § 119 to prior filed U.S. Provisional Patent Application No. 63/233,861 filed on August 17, 2021, which is hereby incorporated by reference as set forth in full herein.

### FIELD

The present application relates generally to catheters, and specifically to sheaths, catheters, and methods of controlling radial orientation thereof.

### BACKGROUND

Deflectable or steerable catheters are used in various medical and surgical procedures, including ablation, such as arrhythmia ablation, mapping, such as cardiac mapping, and drug delivery, such as intracardial drug delivery. In some cases, an orientation of a catheter tube with respect to certain components therein is important for functionality. For example, sensors on a surface of the catheter must often be oriented in a particular way to optimize their function in a navigation system.

In the related art, multiple round lumens and corresponding internal components have been used to control an outer-surface orientation. However, this related art approach requires that multiple internal components be used to maintain or manipulate the orientation of the catheter. This can increase both the size of the catheter and the complexity of operation thereof. Additionally, excessive bending of the flexible circuits in the wrong direction (e.g., on an inner portion of a curve), may damage the flexible circuits over time.

Thus, catheters capable of improved control of radial orientation are desired in the art.

### SUMMARY

A catheter is presented herein, which includes an elongated body sized to traverse vasculature, defining a longitudinal axis, and including: an outer surface; and a proximal end and a distal end disposed on opposite sides thereof; an orientation lumen formed in the elongated body and defining a curved path extending proximally from the distal end towards the proximal end and about the longitudinal axis; an orientation insert disposed within the orientation lumen, the elongated body being rotationally restricted about the longitudinal axis relative to the orientation insert, the orientation insert attached to the distal end of the elongated body configured to deflect the distal end to form a curve; and a flexible circuit disposed on the outer surface of the elongated body, the elongated body being substantially rotationally fixed about the longitudinal axis relative to the orientation insert such that the flexible circuit is disposed toward an outer bend of the curve when the orientation insert deflects the distal end.

The catheter may have a delivery stage and a deployed stage. The orientation insert may be operable to transition the catheter from the delivery stage to the deployed stage.

In the deployed stage, the distal end of the elongated body may be curved forming a generally circular shape.

The elongated body may be radially rotatable in accordance with a radial rotation of the orientation insert.

The catheter may further include a component lumen formed in the elongated body and defining a second path extending proximally from the distal end towards the proximal end.

The component lumen may be a substantially cylindrical geometry.

The orientation lumen may include: a standard portion substantially rotatable about a first portion of the orientation insert; and an orientation portion substantially rotationally fixed about a second portion of the orientation insert.

The second portion may be disposed at the distal end of the elongated body.

The first portion may include a substantially cylindrical geometry.

A cross-section of the orientation lumen may substantially conform to at least one from among a square, a rectangle, a triangle, an oval, a semi-circle, circular with one or more notches, circular with one or more protrusions, and lobular.

The orientation insert may be press fit into the orientation lumen.

The orientation insert may be connected to the elongated body via an adhesive.

A cross-section of the orientation insert may substantially conform to at least one from among a square, a rectangle, a triangle, an oval, a semi-circle, circular with one or more notches, circular with one or more protrusions, and lobular.

The elongated body may be at least one from among swaged, crimped, and clamped to the orientation insert.

The elongated body may include a flexible portion on which the flexible circuit is disposed. The orientation insert may be operable to deflect the flexible portion.

The elongated body may be substantially rotationally fixed about the orientation insert such that the flexible circuit is positioned on an outward bend of a deflection of the flexible portion.

The orientation insert may include a nitinol wire configured to deflects the distal end to form a curve.

The orientation insert may include an oriented pull-wire such that the flexible circuit is disposed toward an outer bend of the curve when the oriented pull-wire is tightened.

According to aspects of the present disclosure, there is provided a navigable sheath including: an elongated body sized to traverse vasculature, defining a longitudinal axis, and including: an outer surface, a proximal end and a distal end disposed on opposite sides thereof, and a shaft extending distally from the proximal end to the distal end; an orientation lumen formed in the elongated body along the shaft and defining a curved path extending proximally from the distal end towards the proximal end and about the longitudinal axis, the elongated body being substantially rotationally fixable about an orientation insert disposed within the orientation lumen, the orientation insert attached to the distal end of the elongated body configured to deflect the distal end to form a curve; and a flexible circuit disposed on the outer surface of the elongated body, the elongated body being substantially rotationally fixed about the longitudinal axis relative to the orientation insert such that the flexible circuit is disposed toward an outer bend of the curve when the orientation insert deflects the distal end.

The navigable sheath may have a delivery stage and a deployed stage. The orientation insert may be operable to transition the navigable sheath from the delivery stage to the deployed stage.

In the deployed stage, the distal end of the elongated body may be curved forming a generally circular shape.

The elongated body may be radially rotatable in accordance with a radial rotation of the orientation insert.

The navigable sheath may further include a component lumen formed in the elongated body and defining a second path extending proximally from the distal end towards the proximal end.

The component lumen may be a substantially cylindrical geometry.

The orientation lumen may include: a standard portion substantially rotatable about a first portion of the orientation insert; and an orientation portion substantially rotationally fixed about a second portion of the orientation insert.

The second portion may be disposed at the distal end of the elongated body.

The first portion may include a substantially cylindrical geometry.

A cross-section of the orientation lumen may substantially conform to at least one from among a square, a rectangle, a triangle, an oval, a semi-circle, circular with one or more notches, circular with one or more protrusions, and lobular.

The orientation insert may be press fit into the orientation lumen.

The orientation insert may be connected to the elongated body via an adhesive.

A cross-section of the orientation insert may substantially conform to at least one from among a square, a rectangle, a triangle, an oval, a semi-circle, circular with one or more notches, circular with one or more protrusions, and lobular.

The elongated body may be at least one from among swaged, crimped, and clamped to the orientation insert.

The elongated body may include a flexible portion on which the flexible circuit is disposed. The orientation insert may be operable to deflect the flexible portion.

The elongated body may be substantially rotationally fixed about the orientation insert such that the flexible circuit is positioned on an outward bend of a deflection of the flexible portion.

The orientation insert may include a nitinol wire configured to deflects the distal end to form a curve.

The orientation insert may include an oriented pull-wire such that the flexible circuit is disposed toward an outer bend of the curve when the oriented pull-wire is tightened.

According to aspects of the present disclosure, there is provided a method of using a using a catheter in a patient, the method including: inserting into the patient a distal end of a catheter, the catheter including: a distal shaft extending proximally from the distal end and defining a longitudinal axis; an orientation lumen connected to the distal shaft and defining a curved path extending proximally from the distal end towards a proximal end of the catheter and about the longitudinal axis; an orientation insert disposed within the orientation lumen, the distal shaft being substantially rotationally fixed to the orientation insert, the orientation insert being attached to the distal end of the catheter such the orientation insert is operable to deflect the distal end to form a curve; and a flexible circuit disposed on an outer surface of the distal shaft, the distal shaft being substantially rotationally fixed about the longitudinal axis relative to the orientation insert pull-wire; and operating the orientation insert to deflect the distal shaft to form a curve while substantially maintaining a rotational orientation of the orientation insert and the distal shaft such that the flexible circuit is disposed toward an outer bend of the curve.

The catheter may have a delivery stage and a deployed stage. Operating the orientation insert may transition the catheter from the delivery stage to the deployed stage.

In the deployed stage, the distal end may be curved forming a generally circular shape.

The distal end may be radially rotatable in accordance with a radial rotation of the orientation insert.

The catheter may further include a component lumen formed and defining a second path extending proximally from the distal end towards the proximal end.

The component lumen may be a substantially cylindrical geometry.

The orientation lumen may include: a standard portion substantially rotatable about a first portion of the orientation insert; and an orientation portion substantially rotationally fixed about a second portion of the orientation insert.

The second portion may be disposed at the distal end.

The first portion may include a substantially cylindrical geometry.

A cross-section of the orientation lumen may substantially conform to at least one from among a square, a rectangle, a triangle, an oval, a semi-circle, circular with one or more notches, circular with one or more protrusions, and lobular.

The orientation insert may be press fit into the orientation lumen.

The orientation insert may be connected to the distal end via an adhesive.

A cross-section of the orientation insert may substantially conform to at least one from among a square, a rectangle, a triangle, an oval, a semi-circle, circular with one or more notches, circular with one or more protrusions, and lobular.

The distal end may be at least one from among swaged, crimped, and clamped to the orientation insert.

The distal end may include a flexible portion on which the flexible circuit is disposed. The orientation insert may be operable to deflect the flexible portion.

The distal end may be substantially rotationally fixed about the orientation insert such that the flexible circuit is positioned on an outward bend of a deflection of the flexible portion.

The orientation insert may include a nitinol wire configured to deflects the distal end to form a curve.

Operating the nitinol wire may include extending the distal end from a sheath or enveloping catheter.

Operating the nitinol wire may include retracting a sheath or enveloping catheter from the distal end.

The orientation insert may include an oriented pull-wire.

Operating the oriented pull-wire may include tightening the oriented pull-wire.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1 illustrates an example catheter according to aspects of the present invention;
Figures 2A-2C illustrate an example lumen, orientation insert, and combined lumen and orientation insert according to aspects of the present disclosure;
Figures 3A and 3B illustrate rotational-orientation maintenance according to aspects of the present disclosure.
Figure 4 illustrates an example lumen and orientation insert configuration according to aspects of the present disclosure;
Figure 5 illustrates example lumen cross-sections according to aspects of the present disclosure;
Figure 6 illustrates example orientation inserts according to aspects of the present disclosure;
Figure 7 illustrates example cross-sections of a catheter with an orientation insert according to aspects of the present disclosure;
Figure 8 is a flowchart of a method of using a catheter according to aspects of the present disclosure;
Figure 9 is a flowchart of a method of creating a catheter according to aspects of the present disclosure;
Figure 10 is an illustration of a treatment incorporating an example catheter according to aspects of the present disclosure.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 99%.

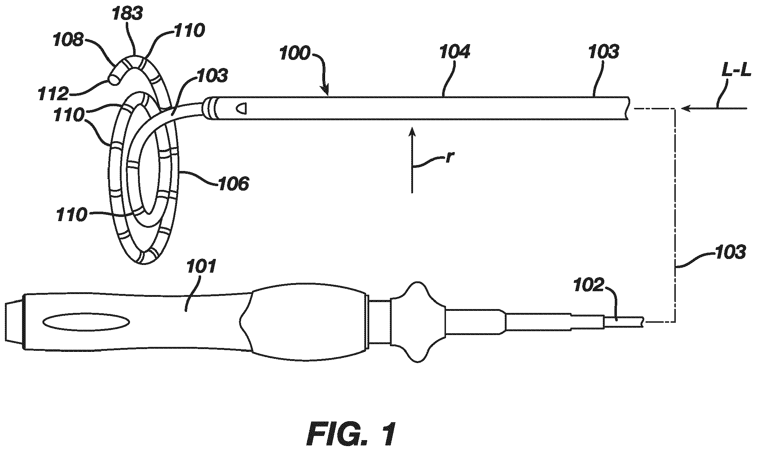

Figure 1 illustrates an example catheter 100, which includes flexible circuits 110 (e.g., flexible sensors 110) disposed on an outer surface of a catheter body 100 (specifically to a cylindrical surface 183 of a distal shaft 106 of catheter 100). The flexible circuits 110 can be fabricated on a flexible circuit 110 and affixed to the cylindrical surface 183. The sensors 110 can include mapping electrodes 110 and can be positioned and otherwise configured to measure electrical signals from tissue in contact with the distal shaft 106.

Figure 1 illustrates the distal shaft 106 of the catheter 100 in a generally circular shape, for example, when within the vasculature or a heart. The generally circular shape can be slightly helical ("lasso"). The flexible circuits 110 can be positioned around the circular shape such that a position and orientation of the distal shaft 106 can be determined in three dimensions when the distal shaft 106 is within a known varying magnetic field. The flexible circuits 110 may be aligned along a longitudinal axis L-L with an oriented radial direction r. The cylindrical surface 183 of the distal shaft 106 curves around the longitudinal axis when the elongated body 103 is in the delivery configuration. The flexible circuits 110 may each conform to the curvature of the cylindrical surface. An ablation electrode 112 can be disposed on the distal end 108.

The catheter 100 can include a control handle 101 affixed to a proximal end 102 of the elongated body 103 that can be moved to push the elongated body 103 distally through vasculature. In some examples, the control handle 101 can also be used to move the distal shaft 106 from the delivery configuration to the deployed configuration and vice versa, similar to a multifunctional catheter handle and corresponding catheter as disclosed in U.S. Patent No. 6,987,995 which is hereby incorporated by reference in its entirety into this application, or an alternative system capable of causing the distal shaft 106 to move to an expanded deployed shape.

As illustrated in Figure 1, the distal shaft 106 has a generally circular shape. The circular shape can be generally orthogonal to the longitudinal axis L-L defined by the proximal shaft 104 of the tubular body. Alternatively, the circular shape can be aligned to the longitudinal axis L-L or at an oblique angle to the longitudinal axis L-L. As illustrated, the distal shaft 106 forms a lasso shape that is generally orthogonal to the longitudinal axis L-L of the proximal shaft 104. Regardless of the angle of the circular shape to the longitudinal axis L-L, when the distal shaft 106 is in the generally circular shape, the flexible circuits 110 can be spaced approximately equidistant from each other around the generally circular shape. The distal shaft 106 may include a flexible portion on which the flexible circuits 110 are disposed.

The proximal shaft 104 can have an elongated tubular construction. The proximal shaft 104 can have a single, axial, or central lumen. The proximal shaft 104 can be flexible, i.e., bendable, but substantially non-compressible along its length. The proximal shaft 104 can be of any suitable construction and made of any suitable material. In some examples, the proximal shaft 104 has an outer polymer wall having an interior braided metal mesh. The proximal shaft 104 can have sufficient structural integrity such that when the control handle 101 is rotated, the tubular body 103, including the proximal shaft 104 and distal shaft 106, rotate in a corresponding manner. The outer diameter of the proximal shaft 104 is preferably about 7 French or about 8 French.

The useful length of the catheter 100, i.e., that portion that can be inserted into the body, can vary as appropriate based on treatment procedure and anatomy of a patient. For most treatments, the useful length can be about 120 centimeters (cm) to about 181 cm. The length of the distal shaft 106 is a relatively small portion of the useful length and preferably is about 3.5 cm to about 10 cm, and more preferably about 5 cm to about 6.5 cm.

In some examples, the distal shaft 106 can have a section aligned with the longitudinal axis L-L, when the distal shaft 106 is in a substantially circular shape, measuring about 3 millimeters (mm) to about 12 mm. The distal end 108 of the tubular body 103 may or may not overlap the distal shaft 106 when in the circular shape. The generally circular shape can have a circumference measuring approximately equal to the length of the distal shaft 106, one half the length of the distal shaft 106, or may deviate from the length of the distal shaft 106 to a greater or lesser extent. Further, in some examples, the circumference of the circular shape can be modified in-patient via manipulation of the control handle 101. The circular shape can have a circumference measuring about 3 cm to about 8 cm, more preferably about 4 cm to about 6 cm, and more preferably about 5 cm.

The proximal shaft 104 and distal shaft 106 can be joined with glue or the like through a transition zone/region/point. In some examples, the junction can include a spacer similar to the one as described in U.S. Patent No. 5,964,757 which is hereby incorporated by reference in its entirety into this application.

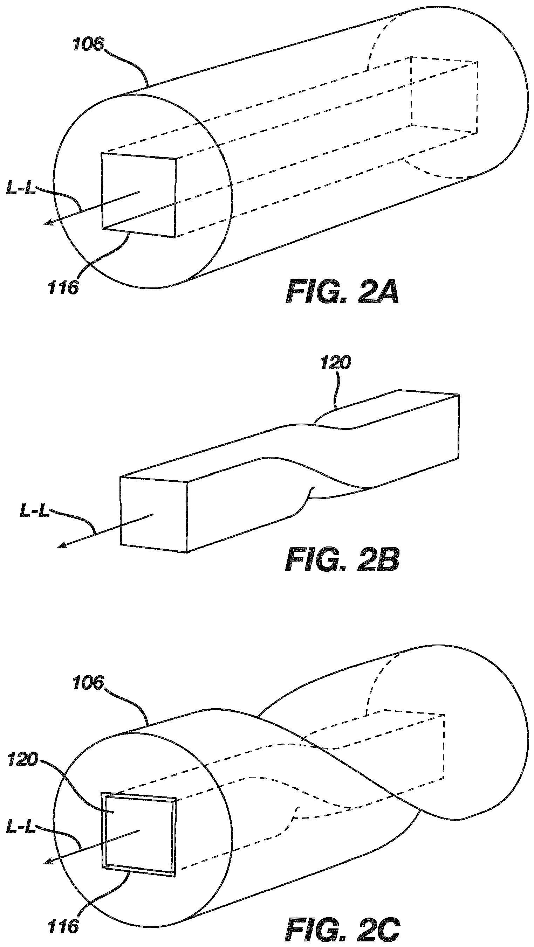

Figures 2A-2C illustrate an example distal shaft 106, orientation insert 120, and combined distal shaft 106 and orientation insert 120 according to aspects of the present disclosure. As can be seen, distal shaft 106 may include a lumen 116 formed therein. Orientation insert 120 may be, for example, an oriented pull-wire 120 attached to the distal end 108 of the distal shaft 106. However, this is just an example. In some cases, orientation insert 120 may be, for example, a nitinol wire 120 configured to control a shape of the distal shaft 106. For instance, when the distal shaft 106 is pushed out from a sheath or housing catheter, the nitinol wire 120 can spring the distal shaft 106 into a predetermined lasso shape.

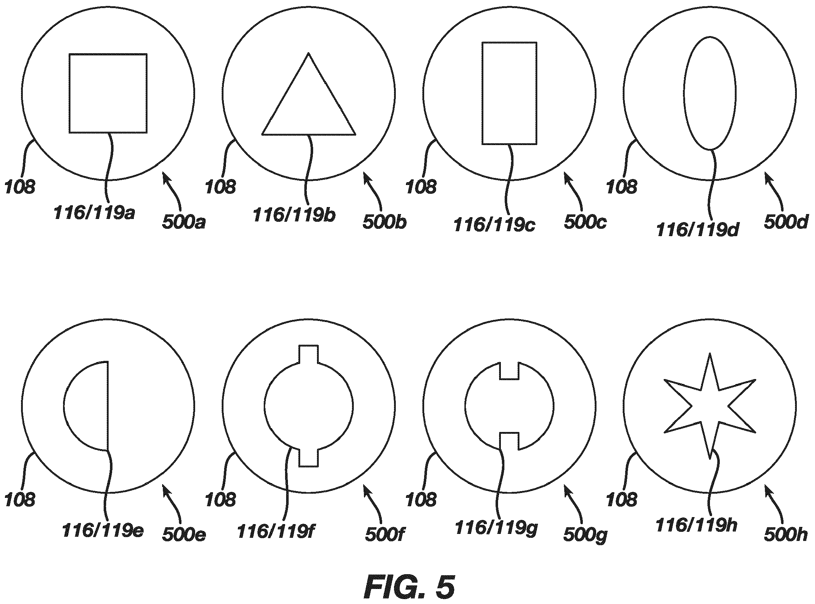

As can be seen, instead of having circular or cylindrical geometry, orientation insert 120 and lumen 116 may have differing shapes (e.g., square). The different shapes may make distal shaft 106 be rotationally restricted about the longitudinal axis L-L to the orientation insert 120. The distal shaft 106 may be substantially rotationally fixed about the longitudinal axis L-L to the orientation insert 120. One of ordinary skill will understand that this is merely an example, and various shapes (for example, as discussed with reference to Figures 5-7) and/or methods (such as press-fit, adhesive, swaging, crimping, or clamping to the orientation insert) may be used to rotationally restrict distal shaft 106 about orientation insert 120.

The distal shaft 106 may be radially rotatable in accordance with a radial rotation of the orientation insert 120. The orientation insert 120 may be operable to transition the distal shaft 106 from the delivery configuration to the deployed configuration. For example, tightening the oriented pull-wire 120 may deflect the distal shaft 106 to form a curve, while maintaining a relative orientation of the distal shaft 106 relative to orientation pull-wire 120.

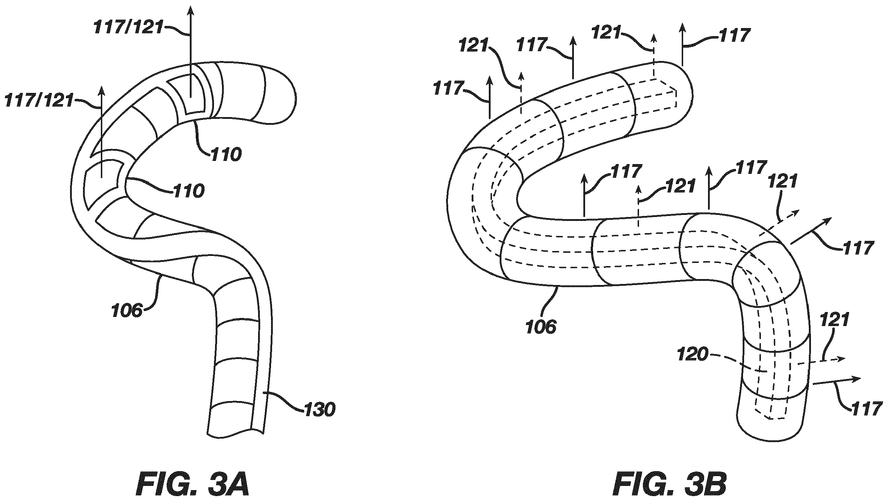

Figures 3A and 3B illustrate rotational-orientation maintenance according to aspects of the present disclosure. In Figures 3A and 3B, a distal shaft 106 had embedded therein an orientation insert 120. Circuits 110 are disposed on distal shaft 106 oriented in radial direction 117. An orientation 121 of the orientation insert 120 is matched to orientation 117, such that the relative orientation of distal shaft 106, orientation insert 120, and flexible circuits 110 are maintained. Furthermore, a flexible circuit line 130 for flexible circuits 110 may be maintained on an outside bend of the distal shaft 106 to reduce compression stress (e.g., improving the operable life and reliability of the catheter 100).

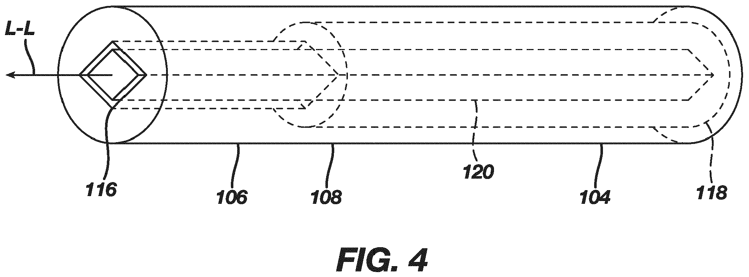

Figure 4 illustrates an example lumen according to aspects of the present disclosure. In Figure 4, distal shaft 106 defines an orientation lumen 116 that extends from a standard lumen 118 formed in proximal shaft 104. Thus, orientation lumen 116 and standard lumen 118 may be thought of as a single lumen 116/118 with an orientation portion 116 and a standard portion 118. The orientation lumen 116 may be rotationally restricted about orientation insert 120, for example, as discussed herein. However, standard lumen 118 may be substantially rotationally independent from an orientation or orientation insert 120. For example, standard lumen 118 may have a substantially circular cross-section, which orientation lumen 116 may have a shaped cross-section (e.g., square). As such, a shaped orientation insert 120 may control the orientation of distal shaft 106, while being largely agnostic to the orientation of proximal shaft 104.

Figure 5 illustrates example lumen cross-sections 119a-119h according to aspects of the present disclosure. For example, lumen cross-sections 119a-119h of orientation lumens 116 may be formed within a distal shaft 106 (e.g., at distal end 108). As depicted in Figure 5, lumen 116 may have a cross-section 119a-119h that is substantially square 500a/119a, triangular 500b/119b, rectangular 500c/119c, ovular 500d/119d semi-circular 500e/119e, circular with one or more notches 500f/119f, circular with one or more protrusions 500g/119g, or lobular (e.g., hexalobular) 500h/119h. However, these are merely examples, and one of ordinary skill will recognize that lumen 116 may have various differing cross-sections without departing from the scope of the present disclosure.

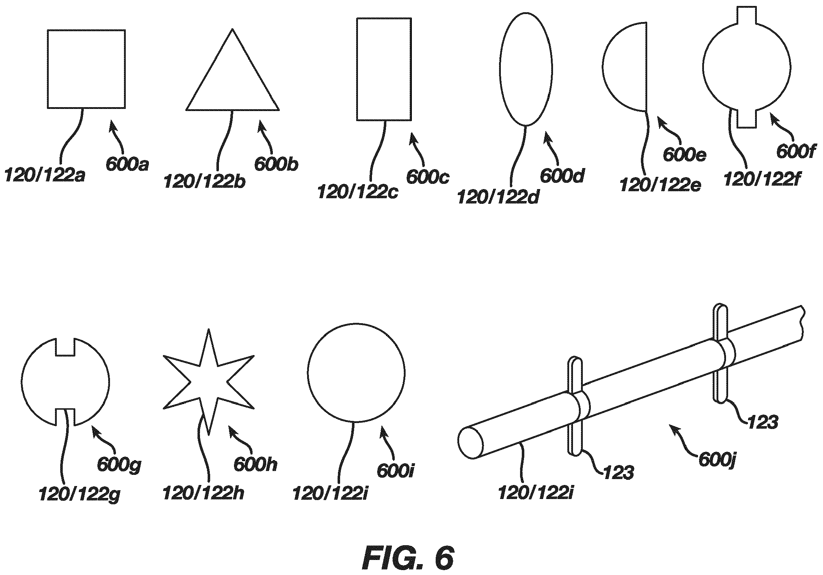

Figure 6 illustrates example orientation inserts 120 according to aspects of the present disclosure. For example, cross-sections 122a-122i of orientation insert 120 may be formed to mate with a shape of a distal shaft 106. As depicted in Figure 6, the orientation insert 120 may have a cross-section 122a-122i that is substantially square 600a/122a, triangular 600b/122b, rectangular 600c/122c, ovular 600d/122d semi-circular 600e/122e, circular with one or more notches 600f/122f, circular with one or more protrusions 600g/122g, or lobular (e.g., hexalobular) 600h/122h. However, these are merely examples, and one of ordinary skill will recognize that lumen 116 may have various differing cross-sections without departing from the scope of the present disclosure. For example, in some cases, orientation insert 120 may have a substantially circular cross-section 600i/122i. In such cases, additional methods (such as press-fit, adhesive, swaging, crimping, or clamping to the orientation insert) may be used to rotationally restrict distal shaft 106 about orientation insert 120. In some cases, external features 123 (e.g., keys) may be added to orientation insert 120 to rotationally restrict distal shaft 106 to orientation insert 120.

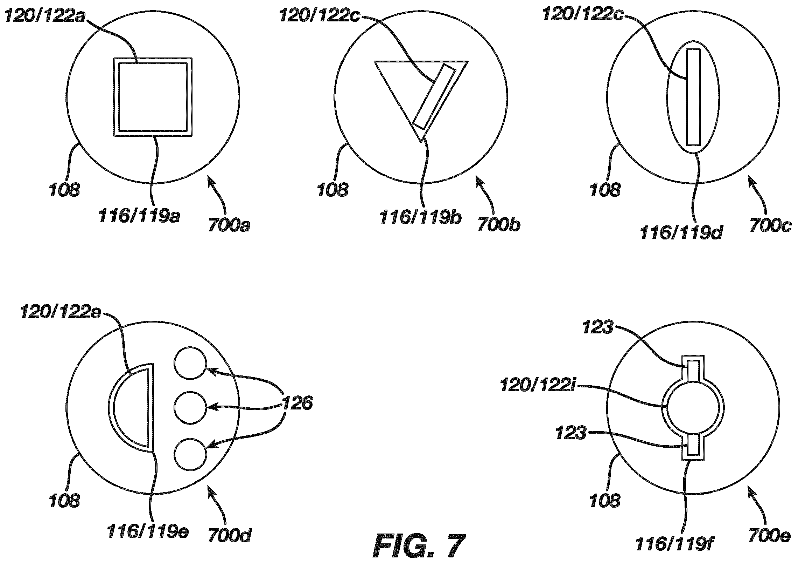

Figure 7 illustrates example cross-sections of a catheter 100 with an orientation insert 120 according to aspects of the present disclosure. As depicted in Figure 7, a cross-section 122 of orientation insert 120 may match a cross-section 119 of lumen 116. For example, lumen 116 and orientation insert 120 may each have a substantially square cross-sections 700a/119a/122a, or lumen 116 and orientation insert 120 may each have a substantially semi-circular cross-sections 700d/119e/122e. However, this is merely an example, and, in some cases, a cross-section 122 of orientation insert 120 may differ from a cross-section 119 of lumen 116. For example, lumen 116 may have a triangular cross-section 119b, while orientation insert 120 may have a substantially rectangular insert 122c 700b. Likewise, lumen 116 may have an ovular cross-section 119d, while orientation insert 120 may have a substantially rectangular insert 122c 700c. In such cases, distal shaft 106 may have greater freedom of movement relative to orientation insert 120 than where the cross-sections match. Additionally, in some cases, lumen 116 may have a cross-section that is circular with one or more protrusions 119f, while external features 123 (e.g., keys) may be added to orientation insert 120 to rotationally restrict distal shaft 106 to orientation insert 120 233i 700e. In some cases, one or more additional component lumens 126 may be formed in the distal shaft. The component lumen 126 may be used to deliver or embed differing elements to the distal end 108. In some cases, component lumens 126 may be substantially circular.

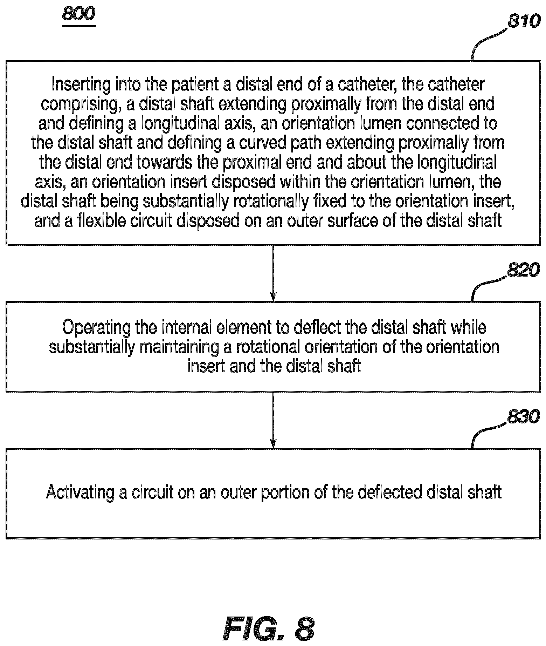

Figure 8 is a flowchart of a method 800 of using a catheter 100 according to aspects of the present disclosure. The method may be performed, for example, by a medical professional. The method may include inserting 810 the distal shaft 106 of a catheter 100 into a patient. The method could include inserting 810 a distal end 108 of a catheter 100 into a patient. The catheter 100 includes an orientation lumen 116 formed in the distal shaft 106 and defines a curved path extending proximally from the distal end 108 towards the proximal end 102 and about the longitudinal axis. The orientation insert 120 may be disposed within the orientation lumen 116. The distal shaft 106 may be substantially rotationally fixed to the orientation insert 120, and a flexible circuit 110 may be disposed on an outer surface of the distal shaft.

Once in the desired position, the method may further include operating 820 the orientation insert 120 to deflect the distal shaft 106 while substantially maintaining a rotational orientation of the internal element and the distal shaft. This may transition catheter 100 from a delivery configuration to a deployed configuration (e.g., lasso). The method 800 may then include activating 830 the flexible circuit 110 that is oriented on an outer portion of the deflected distal shaft.

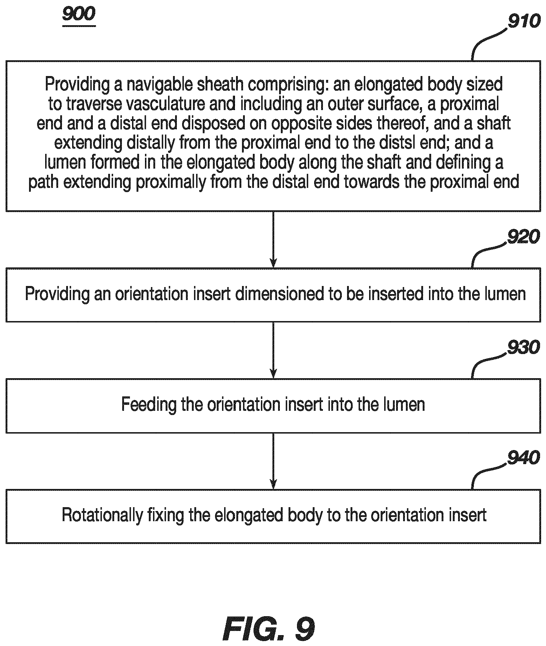

Figure 9 is a flowchart of a method 900 of creating a catheter according to aspects of the present disclosure and according to one or more examples above. Referring to Figure 9, the method 900 may include providing 910 a navigable sheath. The navigable sheath may include, for example, an elongated body 103 (e.g., distal shaft 106) sized to traverse vasculature and including an outer surface 183, a proximal end 102 and a distal end 108 disposed on opposite sides thereof, and a shaft extending distally from the proximal end 102 to the distal end 108. A lumen 116 may be formed in the elongated body 103 along the shaft 106 and define a path extending proximally from the distal end towards the proximal end 102.

The method 900 may further include providing 920 an orientation insert 120 dimensioned to be inserted into the lumen 116. The orientation insert 120 may be a similar shape to lumen 116. The orientation insert 120 may then be fed 930 into the lumen 116. Finally, the elongated body 103 (e.g., distal shaft 106) may be rotationally fixed 940 to the orientation insert 120. In some cases, rotationally fixing 940 may be accomplished by shaped mating of lumen 116 and orientation insert. However, this is merely an example, and, in some cases, other methods (such as, press-fit, adhesive, swaging, crimping, or clamping to the orientation insert) may be used to rotationally restrict distal shaft 106 about orientation insert 120.

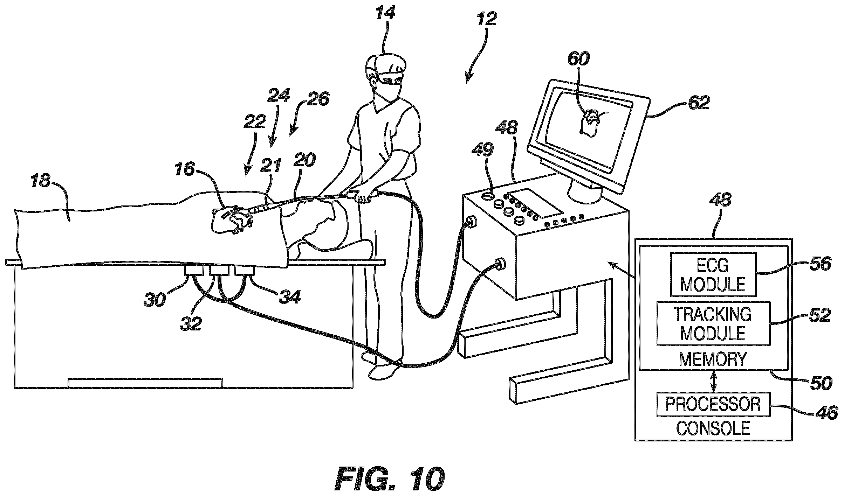

Figure 10 is an illustration of a medical treatment with an example system 12 incorporating an example catheter 100, which can be configured similarly to the example catheters 100 illustrated herein, disclosed herein, or a variation thereof as understood by a person skilled in the pertinent art according to the teachings herein. The treatment is performed by a medical professional 14, and, by way of example, the procedure in the description hereinbelow is assumed to include an investigation of electropotentials a portion of a myocardium 16 of the heart of a human patient 18. However, example catheters 100 can be used in other medical treatment procedures as understood by a person skilled in the pertinent art.

In order to perform the investigation, the professional 14 inserts the catheter 100 into a sheath 21 that has been pre-positioned in a lumen of the patient. The sheath 21 is positioned so that the distal shaft 106 of the catheter 100 enters the heart of the patient 18. The distal shaft 106 include a position sensor 24 including three flexible circuits 110 as illustrated herein (although one of ordinary skill would recognize in light of the present disclosure that position sensor 24 could include various numbers of flexible circuits 110), disclosed herein, or a variation thereof as understood by a person skilled in the pertinent art according to the teachings herein. The position sensor 24 can enable tracking location and orientation of the distal shaft 106 of the catheter 100. The distal shaft 106 can also include mapping electrodes (e.g., flexible circuits 110) or a variation thereof as understood by a person skilled in the pertinent art according to the teachings herein. The mapping electrodes can be used to acquire electropotentials of the myocardium 16.

The position sensor 24 includes flexible circuits 110 which respectively include a plurality of coils. While the description herein describes using the coils for sensing magnetic fields, the coils may also be used to produce magnetic fields.

The system 12 can include a console 48 having a system processor 46. The console 48 can include controls 49 which can be usable by the professional 14 to communicate with the processor 46. The software for the processor 46 can be downloaded to the processor in electronic form, over a network, for example. Alternatively, or additionally, the software can be provided on non-transitory tangible media, such as optical, magnetic, or electronic storage media. Tracking (e.g. position and orientation) of distal shaft 106 of the catheter 100 can be displayed on a three-dimensional representation 60 of the heart of patient 18 that is displayed on a screen 62.

In order to operate the system 12, the processor 46 communicates with a memory 50, which has a number of modules used by the processor 46 to operate the system 12. Thus, the memory 50 can include an electrocardiograph (ECG) module 56 which acquires and analyzes signals from the mapping electrodes (e.g., flexible sensors 110). The memory 50 can also include a tracking module 52, which receives signals from the position sensor 24, and which analyzes the signals in order to generate the location and orientation of distal shaft 106. An ECG module 56 and the tracking module 52 can include hardware and/or software components. The memory 50 can include other software modules, such as a force module for measuring the force on the distal shaft 106, and/or an irrigation module allowing the processor 46 to control irrigation provided for the distal shaft 106. For simplicity, such other modules are not illustrated in Figure 10.

In addition to receiving and analyzing signals from the position sensor 24, the tracking module 52 can also control radiators 30 32, 34. The radiators can be positioned in proximity to myocardium 16 and can be configured to radiate alternating magnetic fields into a region in proximity to the myocardium 16. The position sensor 24 can be configured to produce electrical signals which can be transmitted to the console 48 to be interpreted by the tracking module 52 to determine a three-dimensional position and orientation of the distal shaft 106 of the catheter 100. Each of the flexible circuits 110 can be configured to generate the electrical signals of the position sensor 24 in response to the radiated magnetic fields traversing coils of the flexible circuits 110, thereby enabling the console 48 to track the distal shaft 106. The Carto^{®} system produced by Biosense Webster uses such a magnetic tracking system. When the distal shaft 106 is in a correct position, one or more pull-wires can be activated (e.g., pulled) at the proximal end 102 to deflect the distal shaft 106, for example, in a lasso or loop.

The descriptions contained herein are examples of embodiments of the invention and are not intended in any way to limit the scope of the invention. As described herein, the invention contemplates many variations and modifications of the catheter 100 and methods for manufacturing and using the same. Additional modifications that are apparent to those having skill in the art to which this invention pertains and are intended to be within the scope of the claims which follow.

## Claims

1. A catheter comprising:
an elongated body sized to traverse vasculature, defining a longitudinal axis, and comprising:
an outer surface; and
a proximal end and a distal end disposed on opposite sides thereof;
an orientation lumen formed in the elongated body and defining a curved path extending proximally from the distal end towards the proximal end and about the longitudinal axis;
an orientation insert disposed within the orientation lumen, the elongated body being rotationally restricted about the longitudinal axis relative to the orientation insert, the orientation insert attached to the distal end of the elongated body configured to deflect the distal end to form a curve; and
a flexible circuit disposed on the outer surface of the elongated body, the elongated body being substantially rotationally fixed about the longitudinal axis relative to the orientation insert such that the flexible circuit is disposed toward an outer bend of the curve when the orientation insert deflects the distal end.

2. The catheter of claim 1,
the catheter having a delivery stage and a deployed stage, and
the orientation insert being operable to transition the catheter from the delivery stage to the deployed stage.

3. The catheter of claim 2, wherein in the deployed stage, the distal end of the elongated body is curved forming a generally circular shape.

4. The catheter of claim 1, a cross-section of the orientation insert substantially conforming to at least one from among a square, a rectangle, a triangle, an oval, a semi-circle, circular with one or more notches, circular with one or more protrusions, and lobular.

5. The catheter of claim 1,
the elongated body comprising a flexible portion on which the flexible circuit is disposed, and
the orientation insert being operable to deflect the flexible portion.

6. The catheter of claim 5, the elongated body being substantially rotationally fixed about the orientation insert such that the flexible circuit is positioned on an outward bend of a deflection of the flexible portion.

7. The catheter of claim 1, the orientation insert comprising a nitinol wire configured to deflect the distal end to form a curve.

8. The catheter of claim 1, the orientation insert comprising an oriented pull-wire such that the flexible circuit is disposed toward an outer bend of the curve when the oriented pull-wire is tightened.

9. A navigable sheath comprising:
an elongated body sized to traverse vasculature, defining a longitudinal axis, and comprising:
an outer surface,
a proximal end and a distal end disposed on opposite sides thereof, and
a shaft extending distally from the proximal end to the distal end;
an orientation lumen formed in the elongated body along the shaft and defining a curved path extending proximally from the distal end towards the proximal end and about the longitudinal axis, the elongated body being substantially rotationally fixable about an orientation insert disposed within the orientation lumen, the orientation insert attached to the distal end of the elongated body configured to deflect the distal end to form a curve; and
a flexible circuit disposed on the outer surface of the elongated body, the elongated body being substantially rotationally fixed about the longitudinal axis relative to the orientation insert such that the flexible circuit is disposed toward an outer bend of the curve when the orientation insert deflects the distal end.

10. The navigable sheath of claim 9,
the navigable sheath having a delivery stage and a deployed stage, and
the orientation insert being operable to transition the navigable sheath from the delivery stage to the deployed stage.

11. The navigable sheath of claim 10, in the deployed stage, the distal end of the elongated body being curved forming a generally circular shape.

12. The catheter of claim 1 or the navigable sheath of claim 9, the elongated body being radially rotatable in accordance with a radial rotation of the orientation insert.

13. The catheter of claim 1 or the navigable sheath of claim 9 further comprising a component lumen formed in the elongated body and defining a second path extending proximally from the distal end towards the proximal end.

14. The catheter of claim 1 or the navigable sheath of claim 9, the orientation lumen comprising:
a standard portion substantially rotatable about a first portion of the orientation insert; and
an orientation portion substantially rotationally fixed about a second portion of the orientation insert.

15. The catheter of claim 14, the second portion being disposed at the distal end of the elongated body.

16. The catheter of claim 14, the first portion comprising a substantially cylindrical geometry.

17. The navigable sheath of claim 9, a cross-section of the orientation lumen substantially conforming to at least one from among a square, a rectangle, a triangle, an oval, a semi-circle, circular with one or more notches, circular with one or more protrusions, and lobular.
